# EUROPEAN PATENT APPLICATION

(11) **EP 3 915 398 A1**
(43) Date of publication of application: **01.12.2021**
(21) Application number: 20177558.2
(22) Date of filing: 29.05.2020
(51) Int. Cl.: A23L 33/00, A21D 13/04, A21D 13/045, A21D 13/047, A21D 13/41, A23L 33/20, G06Q 30/00, G06Q 30/02, G09B 19/00, G16H 20/60

(54) **METHOD FOR DETERMINING OPTIMIZED COMPOSITIONS OF FOOD PRODUCTS**

(71) Applicant: Nutriomix GmbH, 88400 Biberach an der Riss (DE)
(72) Inventor: Cayli, Aziz, 10629 Berlin (DE)
(74) Representative: Wohlfahrt, Jan Günther

(57) **Abstract**

The present invention pertains to a method for determining the composition of a food product, a method for preparing a food product, a food product prepared by the method according to the present invention and the use thereof.

## Description

Industry illnesses, such as cardiovascular disease, high blood pressure, diabetes mellitus type 2 (DMT2), some cancers and poor bone health have been shown to have a certain degree of correlation with eating patterns. The proportion of overweight or obese people is alarmingly increasing. Poor eating patterns correspond to excessive consumption of certain nutrients, like saturated fat or sugar, but also to low intake of other important nutrients, like vitamin B6 or potassium.

The potential impact of diet to the health status is enormous when combined with not smoking and regular physical activity. Previous studies have shown that about 82 percent of heart attacks, about 70 percent of strokes, over 90 percent of type 2 diabetes, and over 70 percent of colon cancer, could be prevented with the right dietary choices.

A large variety of diets is available for different purposes, e.g. to lose weight, to gain weight, to build muscle, to treat diabetes, etc. Even though some diets promote expected effects in short term, in longer term almost all of them fail meeting the goals. Therefore, the number of people with nutrition related illnesses increases worldwide. Apparently, the problems associated with poor eating patterns could not be solved. Contrarily, diet related problems are increasing.

One reason for failing diets might relate to the reductionist thinking. In order to understand the problem, scientists often tend to isolate one aspect of the problem and evaluate it isolated from the rest of variables. The reductionist thinking has dominated up to now the diets and the diet related food products. Diets focusing on reduction of carbohydrates, on reduction of fats or on increase of the protein content in food products are typical examples of reductionist thinking. However, it has to be considered that the human body is complex and foods are complex as well. Thus, two complex systems start interacting with each other when food is consumed. Many parameters start to influence each other and to act synergistically. It is impossible to isolate one parameter and try to solve the problem only in light of that single aspect.

Reductionist thinking is illustrated in following example: There are 11 different saturated fatty acids, 6 mono unsaturated fatty acids, 6 poly unsaturated n-6 fatty acids and 5 poly unsaturated n-3 fatty acids published in foods. These fatty acids are the ones which were measurable with the analytical tools today. Certainly, there is a larger number of unknown fatty acids in foods which are not detectable with current analytical tools. Fatty acids of foods are complex and the exact functions of most of the fatty acids are still unknown. Despite this, the scientific world tends to reduce the discussion to omega-3 fatty acids (good fat) and to saturated fatty acids (bad fats). Even though a food consists of thousands of nutrients each with a distinct function, the food is reduced to its omega-3 fatty acid content only. As omega-3 fatty acids were shown to have a positive effect on health, it is recommended to consume foods with omega-3 fatty acids or omega-3 fatty acid pills. In contrast, foods comprising saturated fatty acids are recommended to be avoided.

The problem might be solved partly by holistic approaches. Holistic approaches consider consuming whole foods instead of isolated food parts. There are efforts to consider macronutrients in a diet and to avoid imbalance of macronutrients in foods (US 2019/0159505 A1). Even that approach is only partly holistic as focus is particularly set on macronutrients such as carbohydrates, fats, and proteins. However, these macronutrients merely represent a fraction of the complex cellular requirements. Cells need hundreds or thousands of different nutrients to function optimally. Required salts, trace elements, vitamins all together are not considered in current approaches. Even focusing on a particular macronutrient, e.g. "protein" cannot bring expected positive results. It has to be considered that in case there is an imbalance in the amino acid composition of food due to its protein composition, that food cannot be nutritionally optimal for the body even if the protein concentration itself is optimal. It is therefore necessary to go one further step in detail and to balance at least the amino acid composition, fatty acid composition, vitamin composition and mineral composition in food products.

Different attempts are published to optimize the nutritional profile of foods. Most of these optimization studies focus to caloric intake from carbohydrates, proteins or fats. Current food products are often supplemented with single isolated nutrients, e.g. vitamins, minerals or food fragments like whey or milk protein (WO 01/67889 A1). However, adding single nutrients into a food is a reductionist approach. Balancing the nutritional profile of foods is far more than balancing the caloric intake from macronutrients. Single nutrients are interacting with each other and generate positive or negative synergistic effects in the cells. For example, it has been demonstrated that proteins in combination with vitamin D and calcium together promote muscle formation. In first instance these single nutrients do not belong to the same category of nutrients. Proteins belong to macronutrients and are source of essential and non-essential amino acids. Thus, proteins are source of energy and contribute to regeneration. Vitamin D promotes vitality. Calcium is a mineral that is known to promote bone strength. However, only in combination those three single nutrients generate a synergy with totally different biological functions and promote muscle formation (US 10,455,853 B2). Thus, for optimal body function essential, semi-essential and non-essential nutrients need to be optimized in a similar way because nutrients work in a synergistic way.

Making healthier food choices can help prevent some illnesses. Food-based dietary guidelines, like the Dietary Guidelines for Americans, provide general population recommendations for healthy eating. These tend to be based on nutrient adequacy and reduced risk of diet-related diseases. In this way, dietary guidelines are published for example by Institute of Medicine (IOM) the Dietary Reference Index (DRI) or the Healthy Eating Index (HEI 2015). Such guidelines can drive nutrition education or menu planning. Recommendations for healthier food choices are divided in two levels: at the level of food categories, e.g., eat more fruits, eat less meat, etc. and at the level of nutrients, e.g. eat more omega-3 fatty acids, eat less saturated fatty acids, etc. However, customers have difficulty logistically to organize the food items from different food categories all the time or customer have difficulty to know whether/when nutritional recommended goals have been achieved. These generalized guidelines are difficult to praxis as well since customer need to know the nutrient profile of each food before consumption.

In order to help customers a number of software is available to balance their diets. The software usually recommends to the client certain foods to eat in calculated quantity. Customer record what has been consumed and the program calculates the nutritional balance and suggests the next meal for that specific person (US 2010/0198605 A1). Thus, customer will be monitored over time (WO 2016/050958 A1). Such approach has various drawbacks. First, the approach is customer specific and missing a general applicability. Second, the software suggests to the customer actually a behavioral change in eating habits. Behavior of a person can change in short term and customer might get positive results, but in long term, customer will fall back to its old behavioral patterns, such that the concept is not sustainable. Third, these approaches generate constant logistic stress to search for the specific food suggested by the software. In busy everyday life it is not simple to have the suggested food available at the required moment. Consequently, a person might often have the tendency to eat whatever is available at home and loose the motivation to exactly follow the recommendations. Fourth, people's freedom is restricted to freely choose a food. A person is required choose one of a few alternatives suggested by the software. In this regard, US 2010/0198605 A1 discloses a food selection system.

Some publications represent some nutrients as healthy and the other nutrients as unhealthy. They recommend to reduce or even omit so called "unhealthy" nutrients, e.g. carbohydrates and increase "healthy" nutrients, e.g. proteins in foods. However, the healthiness of a nutrient is a function of its concentration, not of its presence or absence in food. A good example is the current high protein hype. Food industry produces products with higher and higher protein content because of the trend with little scientific evidence. There are up to 70% protein containing food products available in market. Costumers who eat more protein containing food products hope to gain muscles or lose weight. Even though they might achieve that goal in short term and they might get new health problems with their kidneys by detoxification of highly released ammonia or gout through high uric acid concentration in blood or increased body fat produced from excessive protein consumption. Another common approach of food industry to nutritionally balance the foods is to take any standard food and to supplement it with limiting nutrients. For example, muesli is supplemented with chemically synthesized vitamins. In this way, muesli seems to be nutritionally more balanced but this approach has following deficiencies. First, addition of a single nutrient or group of nutrients, e.g. vitamins can have either no or detrimental effect to cell metabolism. The basic idea was to balance the nutritional profile of a food product by supplementing it with an isolated nutrient. In reality, that food might not have an expected positive effect; even worse in some cases the supplementation might cause negative effect to the body. For example, it became a hype to supplement food with antioxidants because of many positive reports on their influence on health. However, a recent study demonstrated that supplementing the foods with strong antioxidants, vitamin E and N-acetyl cysteine increased tumor progression and reduced the survival in mouse (Sayin, et.al., Science Translational Medicine, 2014: Vol. 6, Issue 221, pp. 221). Secondly, a food is not nutritionally balanced if couple of single nutrients are added to the food. All macronutrients and micronutrients need to be perfectly balanced in a perfect ratio to each other. All nutrients in a food work in a synergistic way to exhibit their positive effect. If only one of hundreds of nutrients is imbalanced, the expected positive effect might not occur. Currently, around 30 macronutrients and micronutrients are measured in foods and only a few macronutrients are balanced by food industry. On the other hand, any food consists of more than 30 single nutrients. A food can rather consists of many thousands of single nutrients. Therefore, supplementing a food by one or two single nutrients (e.g. vitamin E or N-acetyl cysteine or proteins) does not solve the balancing problem. In contrast, it might introduce a new, more dramatic imbalance because of its high dosage.

Accordingly, there is no publication about a food product which is balanced in broad context regarding macronutrients and micronutrients using natural raw materials. Published food products are designed considering only a few macronutrients or micronutrients. Published foods are supplied randomly with a few nutrients without taking care of balancing other nutrients or synergies. Published food products for improving health are mostly supplied with chemically synthetized single nutrients, not with naturally occurring nutrients, natural occurring foods or naturally occurring food raw materials.

Thus, there is a need to offer food products to clients, which do not necessitate behavioral changes, which customers can regularly buy in a simple way and which are healthier alternatives to the common food products in market. There is further a need to offer full freedom to clients to choose their preferred food products.

The present invention solves the technical problem by the subject-matter of the independent claims, in particular by providing a method for determining the composition of a food product according to the present invention, the method for preparing a food product according to the present invention, a food product according to the present invention and the use thereof.

The present invention pertains to a method for determining the composition of a food product, comprising the steps:
a) providing at least one database comprising nutrient profiles of raw materials, a target food product and a target nutrient profile of the target food product, wherein the target nutrient profile encompasses nutrient values of at least 6 nutrients,
b) applying an algorithm for determining the type and quantity of different raw materials in the target food product on a group of nutrient profiles of raw materials in the at least one database,
c) providing the determined type and quantity of raw materials of the target food product having a nutrient profile in which the nutrient values of at least 6 nutrients differ from the corresponding nutrient values in the target nutrient profile by at most 30%.

Accordingly, in the method for determining the composition of a food product, at least one database comprising nutrient profiles of raw materials, a target food product and a target nutrient profile of the target food product are provided in step a), wherein the target nutrient profile encompasses nutrient values of at least 6 nutrients. According to the present invention, a target food product and a target nutrient profile of the target food product can be freely chosen according to the specific requirements or individual preferences. Subsequently, an algorithm for determining the type and quantity of different raw materials in the food product is applied on a group of nutrient profiles of raw materials in the at least one database. In this step, the algorithm determines the type of raw materials and the respective quantity of raw materials in a group of raw materials in the at least one database, which are required to obtain the target food product comprising different raw materials and having a total nutrient profile which corresponds to the target nutrient profile, in particular in which the nutrient values of at least 6 nutrients differ from the corresponding nutrient values in the target nutrient profile by at most 30%.The determined type and quantity of raw materials of the food product having the nutritional profile can then be used to prepare a target food product having a nutrient profile corresponding to the target nutrient profile, in particular in which the nutrient values of at least 6 nutrients differ from the corresponding nutrient values in the target nutrient profile by at most 30%.

Preferably, in step a) at least two, preferably at least three, preferably at least four, preferably at least five, databases comprising nutrient profiles of raw materials are provided.

In a preferred embodiment of the present invention, the food product comprises at least two raw materials, preferably at least three raw materials, preferably at least five raw materials, preferably at least six raw materials.

In a further preferred embodiment of the present invention, the nutrient profiles of the raw materials in the at least one database comprise data on the type and quantity of nutrients, in particular micro- and macronutrients, in the raw materials.

In a particularly preferred embodiment of the present invention, the group of nutrient profiles of raw materials encompasses nutrient profiles of all raw materials in the at least one database. Preferably, the group of nutrient profiles of raw materials encompasses nutrient profiles of selected raw materials in the at least one database, preferably of raw materials selected from non-allergic raw materials, meat-free raw materials, raw materials free of animal products, kosher raw materials and/or halal raw materials. Thus, the method according to the present invention allows the selection of a group of certain specific raw materials, which for example meet cultural or religious dietary practices, ethical dietary practices, medical dietary practices and/or specific taste preferences.

In another preferred embodiment of the present invention, the target nutrient profile of the target food product provided in step a) is improved in comparison to the nutrient profile of a common food product corresponding to the target food product. In particular, the target nutrient profile of the target food product provided in step a) has a higher content of nutrients, a more balanced ratio of nutrients and/or a higher diversity of nutrients than common food product corresponding to the target food product.

In a further preferred embodiment of the present invention, the target nutrient profile of the target food product provided in step a) corresponds to the nutrient profile of a common food product corresponding to the target food product, wherein the target food product varies in at least one, preferably at least two, preferably at least three, preferably at least four, preferably at least five, raw material from the common food product.

Preferably, the target nutrient profile of the target food product provided in step a) corresponds to the nutrient profile of a common food product corresponding to the target food product, wherein the quantity of the nutrients in the target food product is altered in comparison to the common food product.

In a preferred embodiment of the present invention, the target nutrient profile of the target food product provided in step a) is a nutrient profile for a specific group of individuals or for a specific application. Preferably, the target nutrient profile of the food product provided in step a) is a nutrient profile for a specific group of individuals. In a further preferred embodiment of the present invention, the target nutrient profile of the food product provided in step a) is a nutrient profile for a specific application.

Preferably, the specific group of individuals is selected from the group consisting of infants, children up to the age of 12 years, adults up to the age of 50 years and adults older than 50 years. Particularly preferred, the specific group of individuals are infants, in particular infants up to the age of 2. In a further preferred embodiment of the present invention, the specific group of individuals are children, in particular children up to the age of 12. Preferably, the specific group of individuals are adults, in particular adults up to the age of 50. Preferably, the specific group of individuals are adults older than 50, preferably adults older than 60, preferably adults older than 70, preferably adults older than 80. In another preferred embodiment of the present invention the specific group of individuals are pregnant women. Preferably, the specific group of individuals are lactating women. In a particularly preferred embodiment of the present invention, the specific group of individuals are overweight individuals, in particular obese individuals. Preferably, the specific group of individuals are individuals who would like to gain muscle mass. In another preferred embodiment of the present invention, the specific group of individuals are individuals suffering from diabetes, cardiovascular diseases, cancer and/or Alzheimer's disease. Preferably, the group of specific individuals are individuals with an increased risk of developing diabetes, cardiovascular diseases, cancer and/or Alzheimer's disease. Accordingly, the method according to the present invention allows to determine the composition of a food product suitable to meet the nutrient requirements of different groups of individuals, in particular depending on for example the stage of life, genetic background, health condition and/or medication. In a particularly preferred embodiment of the present invention, the target nutrient profile of the food product provided in step a) is a nutrient profile for a specific individual.

In another preferred embodiment, the target nutrient profile of the food product provided in step a) is a gender-specific nutrient profile.

In a preferred embodiment of the present invention, the target nutrient profile encompasses nutrient values of at least 7 nutrients, preferably at least 8 nutrients, preferably at least 9 nutrients, preferably at least 10 nutrients, preferably at least 11 nutrients, preferably at least 12 nutrients, preferably at least 13 nutrients, preferably at least 14 nutrients, preferably at least 15 nutrients, preferably at least 16 nutrients, preferably at least 17 nutrients, preferably at least 18 nutrients, preferably at least 19 nutrients, preferably at least 20 nutrients, preferably at least 25 nutrients, preferably at least 30 nutrients, preferably at least 35 nutrients, preferably at least 40 nutrients, preferably at least 45 nutrients, preferably at least 50 nutrients.

Preferably, the target nutrient profile of the target food product is a nutrient profile providing at least 20%, preferably at least 25%, preferably at least 30%, preferably at least 35%, preferably at least 40%, preferably at least 45%, preferably at least 50%, preferably at least 55%, preferably at least 60%, preferably at least 65%, preferably at least 70%, preferably at least 75%, preferably at least 80%, preferably at least 85%, preferably at least 90%, preferably at least 95%, preferably 100%, of nutrients to meet the dietary reference intakes (DRI) or the recommended dietary allowances (RDA), in particular to meet the dietary reference intakes (DRI) or the recommended dietary allowances (RDA) of a specific group of individuals.

In a preferred embodiment of the present invention, the raw material is a material from a natural source. Preferably, the raw material is a naturally occurring raw material or a part of naturally occurring raw material. In a further preferred embodiment of the present invention, the raw material has been prepared in an industrial process, in particular is a processed raw material. In another preferred embodiment, the raw material is a synthetic raw material.

In a particularly preferred embodiment of the present invention, the target food product has a nutrient profile in which the nutrient values of at least 6 nutrients, preferably at least 7 nutrients, preferably at least 8 nutrients, preferably at least 9 nutrients, preferably at least 10 nutrients, preferably at least 11 nutrients, preferably at least 12 nutrients, preferably at least 13 nutrients, preferably at least 15 nutrients, preferably at least 16 nutrients, preferably at least 17 nutrients, preferably at least 18 nutrients, preferably at least 19 nutrients, preferably at least 20 nutrients, preferably at least 25 nutrients, preferably at least 30 nutrients, preferably at least 35 nutrients, preferably at least 40 nutrients, preferably at least 45 nutrients, preferably at least 50 nutrients, differ from the corresponding nutrient values in the target nutrient profile provided in step a) by at most 50%, preferably at most 45%, preferably at most 40%, preferably at most 35%, preferably at most 30%, preferably at most 25%, preferably at most 20%, preferably at most 15%, preferably at most 10%, preferably at most 5%.

In another preferred embodiment of the present invention, the target food product has a nutrient profile in which the nutrient values of at most 10 nutrients, preferably at most 9 nutrients, preferably at most 8 nutrients, preferably at most 7 nutrients, preferably at most 6 nutrients, preferably at most 5 nutrients, preferably at most 4 nutrients, preferably at most 3 nutrients, preferably at most 2 nutrients, preferably at most 1 nutrient, differ from the corresponding nutrient values in the target nutrient profile provided in step a) by at least 5%, preferably at least 10%, preferably at least 15%, preferably at least 20%, preferably at least 25%, preferably at least 30%, preferably at least 35%, preferably at least 40%, preferably at least 45%, preferably at least 50%.

Particularly preferred, the target food product has a nutrient profile in which the nutrient values of at most 5 nutrients, preferably at most 4 nutrients, preferably at most 3 nutrients, preferably at most 2 nutrients, preferably at most 1 nutrient, exceed the tolerable upper intake level (UL). Preferably, the nutrient value of no nutrient in the nutrient profile of the target food product exceeds the tolerable upper intake level (UL).

In a preferred embodiment of the present invention, the average deviation of the nutrient values in the nutrient profile of the target food product from the nutrient values in the target nutrient profile is at most 5%, preferably at most 10%, preferably at most 15%, preferably at most 20%, preferably at most 25%, preferably at most 30%, preferably at most 35%, preferably at most 40%.

The present invention also pertains to a method for preparing a food product, comprising the steps:
aa) determining the composition of a food product according to the method for determining the composition of a food product of the present invention,
bb) preparing a food product having the composition determined in step aa).

Accordingly, in step aa) the composition of a food product is determined by the method for determining the composition of a food product of the present invention and in step bb) the thereby obtained information on the type and quantity of raw materials of the target food product is used to prepare a food product having the determined composition.

In a preferred embodiment of the present invention, the food product is a ready-to-eat food product. In another preferred embodiment of the present invention, the food product is an instant food product, such as an instant beverage or a food powder. In a particularly preferred embodiment of the present invention, the food product is selected from pasta, bakery, confectionary and beverages. Preferably, the food product is selected from noodles, bread, flour, pizza, muesli, cookies, ice-cream, cream, gel, sauce, beverage.

The present invention further pertains to a food product preparable, in particular prepared, by the method for preparing a food product according to the present invention.

In a preferred embodiment of the present invention, 5 to 80%, preferably 10 to 70%, preferably 30 to 65%, preferably 45 to 60%, of the total energy in the food product is provided by carbohydrates.

In a further preferred embodiment, at most 15%, preferably at most 10%, preferably at most 5%, preferably at most 1%, of the total energy in the food product is provided by free sugars. Preferably, at most 10%, preferably at most 5%, preferably at most 1%, of the total energy in the food product is provided by added isolated sugars.

Preferably, the food product comprises carbohydrates from at least two, preferably at least three, preferably at least four, different raw materials.

In another preferred embodiment of the present invention, the food product comprises at least one carbohydrate with low glycemic index and/or at least one carbohydrate with high glycemic index. Preferably, the food product according to the present invention comprises at least one slowly digestible carbohydrate and/or at least one quickly digestible carbohydrate. Preferably, the food product comprises at least two, preferably at least three, preferably at least four, different carbohydrates.

In a further preferred embodiment, 5 to 50%, preferably 10 to 40%, preferably 15 to 25% of the total energy in the food product is provided by proteins.

Preferably, the food product comprises protein from at least two, preferably at least three, preferably at least four, different raw materials.

In a preferred embodiment of the present invention, the food product has a non-essential amino acids (NAA) to essential amino acids (EAA) ratio of from 10:1 to 1:2, preferably 5:1 to 1:1, preferably 4.5:1 to 3.5:1, preferably 3.5:1 to 2.5:1. In a further preferred embodiment, the food product has a non-essential amino acids (NAA) to essential amino acids (EAA) ratio of at least 2.5, preferably at least 3, preferably at least 3.5, preferably at least 4, preferably at least 4.5, preferably at least 5. Preferably, the food product has a non-essential amino acids (NAA) to essential amino acids (EAA) ratio of at most 10, preferably at most 8, preferably at most 6, preferably at most 5, preferably at most 4.5, preferably at most 4, preferably at most 3.5, preferably at most 3, preferably at most 2.5, preferably at most 2.

In another preferred embodiment of the present invention, the food product has an alanine or glycine concentration lower than 15 wt.-% based on the total protein content, preferably between 15 wt.-% and 10 wt.-%, preferably between 10 wt.-% and 5 wt.-%, preferably between 5 wt.-% and 2 wt.-%, preferably between 2 wt.-% and 0 wt.-%. Preferably, the food product does not comprise alanine. Preferably, the food product does not comprise glycine.

In a further preferred embodiment of the present invention, at most 6%, preferably at most 4%, preferably at most 2%, of the total energy in the food product is provided by alanine.

In a preferred embodiment of the present invention, at most 6%, preferably at most 4%, preferably at most 2%, of the total energy in the food product is provided by glycine.

In a preferred embodiment of the present invention, the food product comprises at least 30 wt.-% branched chain amino acids (BCAA) based on the total weight of the food product. Preferably, the food product comprises 20 to 30 wt.-%, preferably 10 to 20 wt.-%, preferably 5 to 10 wt.-%, preferably 1 to 5 wt.-%, leucine based on the total weight of the food product.

In a preferred embodiment of the present invention, 1 to 8%, preferably 1.5 to 5.5%, preferably 2.5 to 4.4%, of the total energy in the food product is provided by branched chain amino acids (BCAA).

In a preferred embodiment of the present invention, the food product comprises at least 20 wt.-% leucine (Leu) based on the total weight of the food product. Preferably, the food product comprises 15 to 20 wt.-%, preferably 10 to 15 wt.-%, preferably 5 to 10 wt.-%, preferably 1 to 5 wt.-%, leucine based on the total weight of the food product.

In a preferred embodiment of the present invention, 0.5 to 3%, preferably 0.7 to 2.5%, preferably 1.2 to 2%, of the total energy in the food product is provided by leucine (Leu).

In a preferred embodiment of the present invention, the food product comprises at least 20 wt.-% glutamine (Gln) based on the total weight of the food product. Preferably, the food product comprises 15 to 20 wt.-%, preferably 10 to 15 wt.-%, preferably 5 to 10 wt.-%, preferably 1 to 5 wt.-%, glutamine based on the total weight of the food product.

In a preferred embodiment of the present invention, at most 10%, preferably at most 8%, preferably at most 6%, preferably at most 4%, preferably at most 2%, of the total energy in the food product is provided by glutamine (Gln).

In a preferred embodiment of the present invention, the food product comprises at least 20 wt.-% asparagine (Asn) based on the total weight of the food product. Preferably, the food product comprises 15 to 20 wt.-%, preferably 10 to 15 wt.-%, preferably 5 to 10 wt.-%, preferably 1 to 5 wt.-%, asparagine based on the total weight of the food product.

In a preferred embodiment of the present invention, at most 10%, preferably at most 8%, preferably at most 6%, preferably at most 4%, preferably at most 2%, of the total energy in the food product is provided by asparagine (Asn).

In a preferred embodiment of the present invention, the food product comprises at least 40 wt.-% glutamine (Gln) and asparagine (Asn) based on the total weight of the food product. Preferably, the food product comprises 30 to 40 wt.-%, preferably 20 to 30 wt.-%, preferably 10 to 20 wt.-%, preferably 2 to 10 wt.-%, glutamine and asparagine based on the total weight of the food product.

In a preferred embodiment of the present invention, the food product has a glutamine (Gln) or asparagine (Asn) concentration of lower than 20 wt.-% based on the total protein content, preferably between 20 wt.-% and 15 wt.-%, preferably between 15 wt.-% and 10 wt.-%, preferably between 10 wt.-% and 5 wt.-%, preferably between 5 wt.-% and 0 wt.-%. Preferably, the food product does not comprise glutamine or asparagine.

In a preferred embodiment of the present invention, at most 16%, preferably at most 10%, preferably at most 8%, preferably at most 6%, preferably at most 4%, of the total energy in the food product is provided by glutamine and asparagine.

In a preferred embodiment of the present invention, the food product comprises at least 20 wt.-% glutamate (Glu) based on the total weight of the food product. Preferably, the food product comprises 15 to 20 wt.-%, preferably 10 to 15 wt.-%, preferably 5 to 10 wt.-%, preferably 1 to 5 wt.-%, glutamine based on the total weight of the food product.

In a preferred embodiment of the present invention, 1 to 9%, preferably 2 to 6%, preferably 3 to 5%, of the total energy in the food product is provided by glutamate (Glu).

In a preferred embodiment of the present invention, the food product comprises at least 20 wt.-% aspartate (Asp) based on the total weight of the food product. Preferably, the food product comprises 15 to 20 wt.-%, preferably 10 to 15 wt.-%, preferably 5 to 10 wt.-%, preferably 1 to 5 wt.-%, aspartate based on the total weight of the food product.

In a preferred embodiment of the present invention, 1 to 9%, preferably 2 to 6%, preferably 3 to 5%, of the total energy in the food product is provided by aspartate (Asp).

In a preferred embodiment of the present invention, the food product comprises at least 40 wt.-% glutamate (Glu) and aspartate (Asp) based on the total weight of the food product. Preferably, the food product comprises 30 to 40 wt.-%, preferably 20 to 30 wt.-%, preferably 10 to 20 wt.-%, preferably 2 to 10 wt.-%, glutamate and aspartate based on the total weight of the food product.

In a preferred embodiment of the present invention, the food product has a glutamate (Glu) or aspartate (Asp) concentration of lower than 20 wt.-% based on the total protein content, preferably between 20 wt.-% and 15 wt.-%, preferably between 15 wt.-% and 10 wt.-%, preferably between 10 wt.-% and 5 wt.-%, preferably between 5 wt.-% and 0 wt.-%. Preferably, the food product does not comprise glutamate or aspartate.

In a preferred embodiment of the present invention, at most 16%, preferably at most 12%, preferably at most 8%, preferably at most 6%, preferably at most 4%, of the total energy in the food product is provided by glutamate and aspartate.

Preferably, 5 to 50%, preferably 5 to 40%, preferably 10 to 40%, preferably 8 to 30%, preferably 15 to 30%, preferably 20 to 25%, preferably 10 to 20%, preferably 12 to 18%, of the total energy in the food product is provided by fat.

In a particularly preferred embodiment of the present invention, the food product comprises fat from at least two, preferably at least three, preferably at least four, different raw materials.

In a preferred embodiment of the present invention, the food product comprises at least one polyunsaturated fatty acid (PUFA). Preferably, the at least one polyunsaturated fatty acid is selected from the group consisting of docosahexanoic acid (DHA), eicosapentaenoic acid (EPA), alpha-linolenic acid (ALA), linoleic acid (LA) and arachidonic acid (ARA). Preferably, the food product comprises at least one omega-3 fatty acid. In a particularly preferred embodiment of the present invention the at least one polyunsaturated fatty acid and/or the at least one omega-3 fatty acid is contained in at least one naturally occurring raw material, in particular is derived from algae, sea food, fungus, flaxseeds and/or nuts. Particularly preferred, the at least one polyunsaturated fatty acid and/or the at least one omega-3 fatty acid is derived from at least one oil, in particular from olive oil, flax seed oil, fish oil, almond oil, walnut oil, canola oil, algae oil and/or grape seeds oil. In a further preferred embodiment of the present invention, the food product comprises at least one polyunsaturated fatty acid and/or the at least one omega-3 fatty acid is an encapsulated form.

In a further preferred embodiment, at most 20%, preferably at most 10%, preferably at most 5%, of the total energy in the food product is provided by saturated fatty acids.

In another preferred embodiment of the present invention, 0 to 30%, preferably, 3 to 20%, preferably 5 to 15%, preferably 6 to 11% of the total energy in the food product is provided by polyunsaturated fatty acids (PUFAs).

In a preferred embodiment of the present invention, 0 to 30%, preferably 2 to 20%, preferably 3 to 15%, preferably 4 to 8% of the total energy in the food product is provided by n-6 linoleic acid (18:2).

In a preferred embodiment of the present invention, 0 to 5%, preferably 0.2 to 2%, preferably 0.3 to 1%, preferably 0.4 to 0.8% of the total energy in the food product is provided by n-3 alpha linolenic acid (18:3).

In a preferred embodiment of the present invention, 5 to 80%, preferably 10 to 70%, preferably 30 to 65%, preferably 45 to 60%, of the total energy in the food product is provided by carbohydrates and/or 5 to 50%, preferably 10 to 40%, preferably 15 to 25% of the total energy in the food product is provided by proteins and/or 5 to 40%, preferably 8 to 30%, preferably 10 to 20%, preferably 12 to 18%, of the total energy in the food product is provided by fat.

In a particularly preferred embodiment of the present invention, the food product is non-allergenic, in particular does not comprise gluten, lactose, nuts and/or eggs, vegetarian, vegan, kosher and/or halal. Preferably, the food product is non-allergenic, in particular does not comprise gluten, lactose, nuts and/or eggs. In another preferred embodiment, the food product is vegetarian, in particular does not comprise meat. Preferably, the food product is vegan, in particular does not comprise raw material from an animal source. In a further preferred embodiment, the food product is kosher. Preferably, the food product is halal.

In a preferred embodiment of the present invention, the food product consists of naturally occurring raw materials. Preferably, the food product comprises solely nutrients obtained from a natural source. In a particularly preferred embodiment, the food product does not comprise chemically synthesized nutrients. Preferably, the food product does not comprise added isolated nutrients, in particular does not comprise added isolated vitamins, proteins, carbohydrates, fats, fatty acids, amino acids, nucleotides, antioxidants, trace elements and/or minerals.

In a further preferred embodiment of the present invention, the food product comprises at least one added isolated nutrient. Preferably, the food product comprises at least two, preferably at least three, preferably at least four added isolated nutrients. Preferably, the food product comprises at most one added isolated nutrient. Preferably, the food product comprises at most two, preferably at most three, preferably at most four added isolated nutrients. Particularly preferred, the added isolated nutrient is selected from the group consisting of vitamins, proteins, carbohydrates, fats, fatty acids, amino acids, nucleotides, antioxidants, trace elements and minerals. In a preferred embodiment of the present invention, the food product comprises added isolated vitamin D and/or vitamin B12. In a further preferred embodiment of the present invention, the food product comprises added isolated NaCl.

In another preferred embodiment of the present invention, the food product comprises at least one biologically active raw material selected from the group consisting of green tea, turmeric, ginger, saffron and garlic.

In a further preferred embodiment, the food product comprises at least one organoleptically and/or visually appealing raw material selected from the group consisting of natural colorant, synthetic colorant, natural aroma, synthetic aroma, vanilla, ginger, chocolate, cacao, lemon, beetroot, orange, spinach, carrot and carob bean.

Preferably, the food product according to the present invention comprises at least one, preferably at least two, preferably at least three, preferably at least four, raw material selected from the group consisting of grains, legumes, seeds, nuts, oils, fruits, vegetables, sea food, meat, meat products, milk and milk products.

In a preferred embodiment of the present invention, the food product is suitable for human consumption. In a further preferred embodiment, the food product is suitable for animal consumption.

The present invention further pertains to the food product according to the present invention for use in the prevention and/or treatment of blood glucose related diseases, in particular diabetes, obesity, cardiovascular diseases, Alzheimer's diseases and/or cancer.

In another aspect, the present invention relates to the use of the food product according to the present invention for gaining weight, for losing weight, for gaining muscle mass, for lowering total cholesterol serum level, for lowering the non-HDL cholesterol serum level, for lowering the total cholesterol/HDL ratio and/or for lowering the triglyceride serum level.

In the context of the present invention, the terms "raw material" refers to any comestible substance or any additive which can be used for the preparation of a food product. The term encompasses processed or unprocessed naturally occurring raw materials as well as synthetically prepared raw materials. According to the present invention a "raw material" can be a simple substance, such as a specific plant material, for example fruits or vegetables, or a specific animal product, for example meat, milk or egg, but can also be a complex food itself, for example a bread or noodles. In the latter case the "food product" of the present invention can be a complex meal.

In the context of the present invention, the expression "type of raw materials" is used to designate a specific kind of raw material. Typical examples of "types of raw materials" are whole wheat, chickpeas, potatoes, spinach, tomatoes, chicken, salmon.

The term "quantity of raw materials" as used herein refers to a specific amount of raw material. In the context of the present invention, the "quantity of raw materials" can refer to the absolute and relate amount of "raw material" in a composition of a food product.

The term "food" as used herein means any comestible or injectable entity suitable for nutrition which consists of more than one nutrient. According to the present invention, a "food" can be naturally occurring or a product of an industrial process.

In the context of the present invention, the term "food product" is used to designate any prepared comestible or injectable entity consisting of at least two raw materials. A "food product" as used herein can be solid, semi-solid, or liquid. Typical examples of "food products" are bread, noodles, pizza, muesli, cookies, fruit or vegetable juices, instant food and instant beverages.

In the context of the present invention, the term "target food product" means the "food product" which is desired to be obtained. For example, in case a noodle is selected as the "target food product", the algorithm used in the method according to the present invention considers raw materials which are suitable for use in a noodle. The raw materials are not necessarily required to be raw materials commonly used in the production of noodles but can also include unusual raw materials which entirely or partly substitute common raw materials.

The term "meal" as used herein encompasses entities of at least one food product, preferably at least two food products consumed to satisfy the appetite and/or to satisfy the nutritional requirements of an individual. Usually a "meal" is distinguished from a "snack" in terms of the variety of raw materials, foods and/or food products comprises therein and/or in term of quantity. Typical examples for a "meal" are soups, pizzas, pasta dishes, salad bowls, etc.

In the context of the present invention, the term "algorithm" designates a sequence of well-defined unambiguous computer-implementable instructions to perform a specific task. Starting from an initial state and initial "input", the instructions describe a computation that, when executed, proceed through a finite number of well-defined successive steps, eventually producing an "output" and terminating at a final ending state.

The term "nutrient profile" as used herein refers to the different types and concentrations of nutrients in the target food product. A "target nutrient profile" refers to the a specific predetermined "nutrient profile", namely a specific group of target nutrients in a target concentration. A food product or a raw material having a certain "nutrient profile" or a certain "target nutrient profile" can additionally comprise further nutrients which are not part of the "target nutrient profile". In some embodiments, the "target nutrient profile" can be an optimal nutrient profile for a specific group of individuals or for a specific application. In other embodiments the "target nutrient profile" is a nutrient profile which is improved in comparison to the "nutrient profile" of a common food product, for example improved with regard to the balance of nutrients, the type of nutrients, the amount of nutrients and/or the ratio of nutrients to each other. According to the present invention, the "nutrient profile" of the target food product is characterized in that the nutrient values of at least 6 nutrients differ from the corresponding nutrient values in the "target nutrient profile" by at most 30%.

In the context of the present invention, the term "macronutrient" designates nutrients which are required in large amounts for growth and development of a cell or an organism. Macronutrients may include but are not limited to carbohydrates, fats, proteins, amino acids, salts and water.

The term "micronutrients" as used herein encompasses compounds having a biological function and which are required in only minor or trace amounts. In particular embodiments, the term may include organic and inorganic compounds, such as individual amino acids, nucleotides, fatty acids, vitamins, antioxidants, minerals and trace elements, such as but not limited to copper, iron, zinc, selenium and manganese.

In the context of the present invention, the term "essential amino acids" or in short "EAA" as used herein refers to naturally occurring amino acids which cannot be synthesized by the human body *de novo* at a rate sufficient to meet the nutritional requirements and are therefore required to be taken up by food consumption. "Essential amino acids" for healthy adult humans are histidine, isoleucine, leucine, lysine, methionine, cysteine, phenylalanine, tyrosine, threonine, tryptophan, valine.

The term "semi-essential amino acids" is used in the context of the present invention to designate amino acids which can be synthesized in the human body, wherein the synthesis can be limited under specific physiological or pathophysiological conditions.

In the context of the present invention, the term "non-essential amino acids" or in short "NAA" refers to naturally occurring amino acids which can be synthesized in the human body in sufficient amounts to meet the nutritional requirements. Therefore, "non-essential amino acids" are not necessarily required to be taken up by food consumption. "Non-essential amino acids" for healthy adult humans are alanine, arginine, asparagine, aspartic acid, glutamic acid, glutamine, glycine, proline and serine.

In the context of the present invention, the term "a" is meant to include the meaning of "one" or "one or more".

In the context of the present invention, the term "comprising" preferably has the meaning of "containing" or "including", meaning including the specifically identified elements without excluding the presence of further elements. However, in a preferred embodiment, the term "comprising" is also understood to have the meaning of "consisting essentially of' and in a further preferred embodiment the meaning of "consisting of'.

Further preferred embodiments of the invention are subject of the subclaims.

The present invention is further illustrated by way of the following examples and figures.

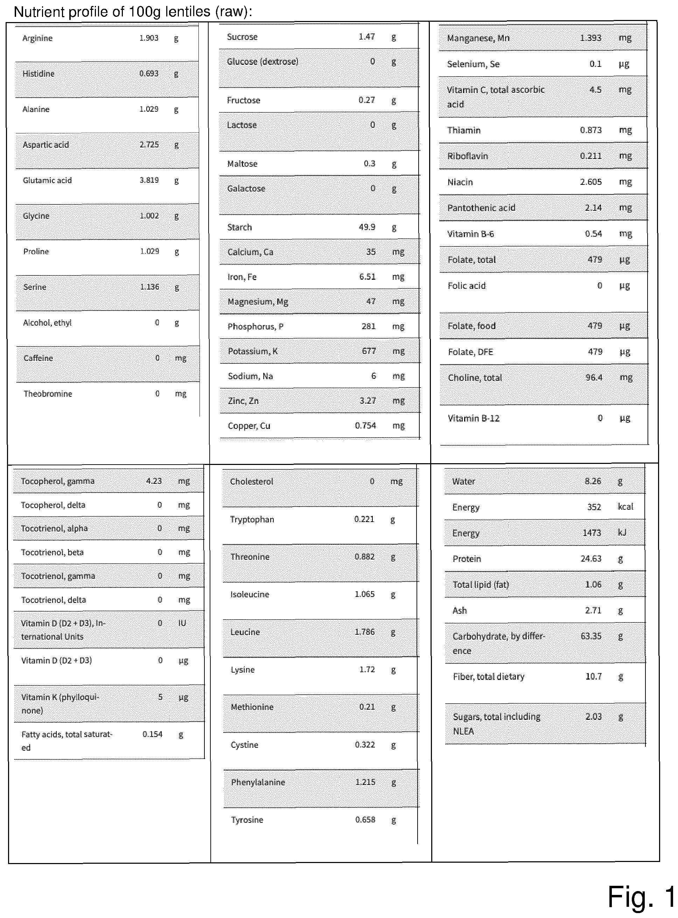

**Figure 1** shows an exemplary nutrient profile of lentils as a raw material obtained from the databank of the U.S. Department of Agriculture on 15 March 2020.

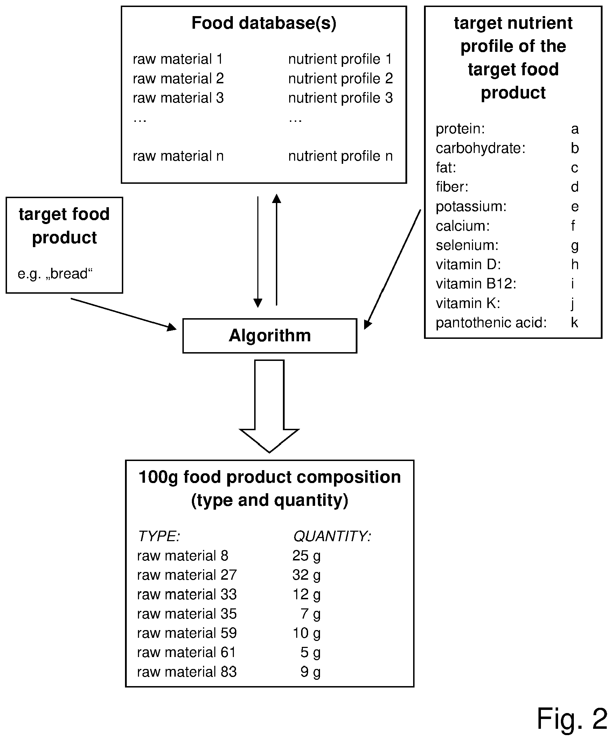

**Figure 2** shows a schematic illustration of the method according to the present invention.

### Example 1: Pizza recipe

Pizza is often considered unhealthy due to a high content of carbohydrates and saturated fats, a low fiber and protein content and a low concentration of polyunsaturated fatty acids. The method according to the present invention has been used to determine an alternative recipe of a pizza having an improved nutrient profile. Table 1 illustrates the ingredients of a traditional pizza.

**Table 1: Ingredients of a traditional pizza.**

| | |
|---|---|
| Wheat, white [g] | 150 |
| Tomatoes, raw [g] | 50 |
| Cheese, cheddar, sharp, sliced [g] | 50 |
| Oil, olive, salad or cooking [g] | 5 |
| Salt, table [g] | 5 |

Based on the ingredients of traditional pizza the nutrient profile is determined. Subsequently, the method according to the present invention is used to determine an alternative composition of pizza having an improved nutrient profile in comparison to traditional pizza. The composition of a pizza determined by the method according to the present invention in comparison to the composition of traditional pizza is given in table 2.

**Table 2: Comparison of ingredients of a traditional pizza and pizza according to the present invention.**

| | Traditional pizza | Pizza according to the present invention Recipe 1: | Pizza according to the present invention Recipe 2: |
|---|---|---|---|
| Wheat, white [g] | 150 | | |
| Wheat, whole [g] | | 75 | 28 |
| Oat [g] | | 75 | 45 |
| Chickpeas [g] | | | 45 |
| Tomatoes, raw [g] | 50 | 50 | 20 |
| Tomatoes, sundried [g] | | | 30 |
| Cheese, cheddar, sharp, | 50 | | |
| sliced [g] | | | |
| Cheese, low fat, cheddar or Colby [g] | | 50 | 45 |
| Oil, olive, salad or cooking [g] | 5 | 1 | |
| Oil, flaxseed, cold pressed [g] | | 4 | |
| Salt, table [g] | 5 | 5 | 5 |
| Seeds, hamp seeds [g] | | | 3 |
| Mushrooms, white [g] | | | 45 |
| Broccoli [g] | | | 24 |
| Leek [g] | | | 10 |
| Spinach [g] | | | 30 |
| Wheat, germ oil [g] | | | 1 |
| Yeast [g\|] | | | 8 |

The nutrient profile of the pizza obtained by the method according to the present invention is improved in comparison to the traditional pizza by about 29% (Recipe 1). Recipe 2 provides an alternative pizza determined by the method according to the present invention having an even more improved nutrient profile in comparison to traditional pizza (62% improvement). Table 3 illustrates exemplary nutritional parameters of three recipes.

**Table 3: Nutritional parameters of traditional pizza and pizza according to the present invention**

| Parameter | Traditional pizza | Pizza according to the present invention Recipe 1: | Pizza according to the present invention Recipe 2: |
|---|---|---|---|
| Fibre [g] | 5 | 17 | 21 |
| Carbohydrate [% of total energy] | 58 | 62 | 62 |
| Protein [% of total energy] | 14 | 21 | 25 |
| Fat [% of total energy] | 26 | 21 | 25 |
| Saturated fat [% of total energy] | 12 | 5 | 5 |
| PUFA [% of total energy] | 2 | 7 | 7 |
| Energy density [calorv/100 g product] | 382 | 326 | 202 |

The recipes for pizza obtained by the method according to the present invention have lower saturated fats, higher fiber content, higher protein content, higher PUFA content and lower calories. In particular the pizza obtained by recipe 2 has a significantly improved nutritional profile in comparison to traditional pizza and is, advantageously, furthermore a low caloric product.

### Example 2: Clinical trials

A clinical study has been performed with a food product obtained by the method according to the present invention. In this study, the glucose to insulin ratio (G:I ratio) was used to measure the effect of the food product on human metabolism. Since the food product obtained by the method according to the present invention reduces the serum insulin level, measurement of the G:I ratio may be used as a simple test for evaluating the therapeutic effectiveness of the food product.

## Claims

1. A method for determining the composition of a food product, comprising the steps:
a) providing at least one database comprising nutrient profiles of raw materials, a target food product and a target nutrient profile of the target food product, wherein the target nutrient profile encompasses nutrient values of at least 6 nutrients,
b) applying an algorithm for determining the type and quantity of different raw materials in the target food product on a group of nutrient profiles of raw materials in the at least one database,
c) providing the determined type and quantity of raw materials of the target food product having a nutrient profile in which the nutrient values of at least 6 nutrients differ from the corresponding nutrient values in the target nutrient profile by at most 30%.

2. The method according to claims 1, wherein the food product comprises at least two raw materials.

3. The method according to claim 1 or 2, wherein the group of nutrient profiles of raw materials encompasses nutrient profiles of selected raw materials in the at least one database, preferably of raw materials selected from non-allergic raw materials, meat-free raw materials, raw materials free of animal products, kosher raw materials and/or halal raw materials.

4. The method according to any one of claims 1 to 3, wherein the target nutrient profile of the food product is a nutrient profile for a specific group of individuals or for a specific application.

5. The method according to claim 4, wherein the target nutrient profile of the food product is a gender-specific nutrient profile.

6. The method according to any one of claims 1 to 5, wherein the raw material is a material from a natural source.

7. A method for preparing a food product, comprising the steps:
aa) determining the composition of a food product according to the method of any of claims 1 to 6,
bb) preparing a food product having the composition determined in step aa).

8. The method according to claim 7, wherein the food product is selected from noodles, bread, flour, pizza, muesli, cookies, ice-cream, cream, gel, sauce, beverage.

9. A food product prepared by the method according to claim 7 or 8.

10. The food product according to claim 9, wherein 5 to 80%, preferably 10 to 70%, preferably 30 to 65%, preferably 45 to 60%, of the total energy in the food product is provided by carbohydrates and/or wherein 5 to 50%, preferably 10 to 40%, preferably 15 to 25% of the total energy in the food product is provided by proteins and/or wherein 5 to 40%, preferably 8 to 30%, preferably 10 to 20%, preferably 12 to 18%, of the total energy in the food product is provided by fat.

11. The food product according to claim 9 or 10, having a non-essential amino acids (NAA) to essential amino acids (EAA) ratio of from 10:1 to 1:2, preferably 5:1 to 1:1, preferably 4.5:1 to 3.5:1, preferably 3.5:1 to 2.5:1.

12. The food product according to any one of claims 9 to 11, having a glutamine or asparagine concentration of lower than 20% of the total protein content, preferably between 20% and 15%, preferably between 15% and 10%, preferably between 10% and 5%, preferably between 5% and 0%.

13. The food product according to any one of claims 9 to 12, having an alanine or glycine concentration lower than 15% of the total protein content, preferably between 15% and 10%, preferably between 10% and 5%, preferably between 5% and 2%, preferably between 2% and 0%.

14. The food product according to any one of claims 9 to 13, wherein the food product is non-allergenic, in particular does not comprise gluten, lactose, nuts and/or eggs, vegetarian, vegan, kosher and/or halal.

15. The food product according to any one of claims 9 to 14 for use in the prevention and/or treatment of blood glucose related diseases, in particular diabetes, obesity, cardiovascular diseases, Alzheimer's diseases and/or cancer.

16. Use of the food product according to any one of claims 9 to 15 for gaining weight, for losing weight, for gaining muscle mass, for lowering total cholesterol serum level, for lowering the non-HDL cholesterol serum level, for lowering the total cholesterol/HDL ratio and/or for lowering the triglyceride serum level.
